(19) Europäisches Patentamt
European Patent Office
Office européen des brevets

(11) Veröffentlichungsnummer : **0 287 846 B1**

(12) # EUROPÄISCHE PATENTSCHRIFT

(45) Veröffentlichungstag der Patentschrift : **19.06.91 Patentblatt 91/25**

(51) Int. Cl.$^5$ : **C07C 229/22**, C07C 255/18, C11D 3/33

(21) Anmeldenummer : **88104891.2**

(22) Anmeldetag : **26.03.88**

(54) **2-Hydroxy-3-amino-propionsäure-N,N-diessigsäure und ihre Derivate, ihre Herstellung und Verwendung insbesondere als Komplexbildner und diese enthaltende Wasch- und Reinigungsmittel.**

(30) Priorität : **11.04.87 DE 3712330**

(43) Veröffentlichungstag der Anmeldung : **26.10.88 Patentblatt 88/43**

(45) Bekanntmachung des Hinweises auf die Patenterteilung : **19.06.91 Patentblatt 91/25**

(84) Benannte Vertragsstaaten : **AT BE CH DE ES FR GB IT LI LU NL SE**

(56) Entgegenhaltungen :
**DE-A- 2 258 257**
**US-A- 3 864 389**
**Chemical Abstracts, Band 54, Nr. 14, 25 Juli 1960, Columbus, Ohio, USA L.Erdey et al, "Preparation of some new complex-forming compounds and determination of their constants" Zusammenfassung-Nr. 14137g-14138b**

(73) Patentinhaber : **BASF Aktiengesellschaft**
**Carl-Bosch-Strasse 38**
**W-6700 Ludwigshafen (DE)**

(72) Erfinder : **Baur, Richard, Dr.**
**Nelkenstrasse 1**
**W-6704 Mutterstadt (DE)**
Erfinder : **Richter, Felix, Dr.**
**Kaiserstrasse 15**
**W-6835 Bruehl (DE)**
Erfinder : **Birnbach, Stefan, Dr.**
**Budapester Strasse 45**
**W-6700 Ludwigshafen (DE)**
Erfinder : **Fikentscher, Rolf, Dr.**
**Von-Stephan-Strasse 27**
**W-6700 Ludwigshafen (DE)**
Erfinder : **Oftring, Alfred, Dr.**
**Berner Weg 26**
**W-6700 Ludwigshafen (DE)**
Erfinder : **Winkler, Ekhard, Dr.**
**Pfalzring 74**
**W-6704 Mutterstadt (DE)**
Erfinder : **Bochnitschek, Werner, Dr.**
**Sinsheimer Strasse 49**
**W-6700 Ludwigshafen (DE)**

EP 0 287 846 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft die 2-Hydroxy-3-amino-propionsäure-N,N-diessigsäure und Derivate, ihre Herstellung und die Verwendung insbesondere als Komplexbildner, Bleichmittelstabilisator und Gerüststoff in Wasch- und Reinigungsmitteln sowie diese enthaltende Wasch- und Reinigungsmittel.

Komplexbildner für Erdalkaliionen und Metallionen, beispielsweise des Calciums, Magnesiums, Eisens, Mangans und Kupfers, werden für die verschiedensten technischen Gebiete benötigt.

Als Einsatzgebiete und Verwendungszwecke kommen beispielsweise in Betracht Wasch- und Reinigungsmittel, technische Anwendungen für Industriereiniger, in der Galvanotechnik, in der Wasserbehandlung und bei Polymerisationen, die photographische Industrie, die Textilindustrie und die Papierindustrie sowie verschiedene Anwendungen in Pharmazeutika, in der Kosmetik, bei Nahrungsmitteln und bei der Pflanzenernährung.

Dem Fachmann geläufige und anerkannte Komplexbildner, insbesondere für Waschmittel, sind beispielsweise Nitrilotriessigsäure (NTA), Ethylendiamintetraessigsäure (EDTA), Ethylendiamintetramethylenphosphonsäure (EDTMP), Propylendiamintetraessigsäure (PDTA), Hydroxypropylendiamintetraessigsäure (HPDTA), Hydroxyethandiphosphonsäure, Diethylentriamintetraessigsäure, Diethylentriamintetramethylenphosphonsäure, Hydroxyethyliminodiessigsäure, Hydroxyethylethylendiamintriessigsäure, Diethylentriaminpentaessigsäure sowie beispielsweise Diethanolglycin, Ethanolglycin, Citronensäure, Glucoheptonsäure oder Weinsäure, wie sie beispielsweise unter dem Stichwort Waschmittel Ullmanns Encyklopädie der technischen Chemie, 4. Aufl., Band 24, S. 63-160, insbesondere S. 91-96, Verlag Chemie, Weinheim, 1983, entnommen werden können.

Die Wirkung der bekannten und teilweise in großem Maßstab eingesetzten Verbindungen ist im Einzelfall nicht immer optimal. Beispielsweise wirkt NTA sehr gut als Komplexbildner und in Wasch- und Reinigungsmitteln recht gut als Gerüststoff zur Verbesserung der Weißwaschwirkung und zur Verhinderung von Ablagerungen, die Inkrustationen und Vergrauung auf dem Gewebe verursachen. Die Wirkung als Bleichmittelstabilisator ist jedoch nur schwach ausgeprägt. Auch EDTA zeigt trotz seines guten Komplexiervermögens gegenüber Schwermetallen eine nur mäßige Wirkung als Bleichmittelstabilisator in Wasch- und Reinigungsmitteln.

In manchen Fällen läßt auch die biologische Abbaubarkeit zu wünschen übrig. So erweist sich EDTA in den üblichen Tests als unzureichend biologisch abbaubar, ebenso PDTA oder HPDTA sowie entsprechende Aminomethylenphosphonate, die zudem wegen ihres Phosphorgehaltes oft unerwünscht sind.

In einer Veröffentlichung von L. Erdey et al. in Acta Chim. Hung., Tomus 21, S. 327-332 (1959) werden die komplexbildenden Eigenschaften von 2,3-Dihydroxypropylamin-N,N-diessigsäure, Serin-N,N-diessigsäure, hergestellt aus D,L-Serin und Chloressigsäure, und L-Gutaminsäure-N,N-diessigsäure im Hinblick auf die Stabilität der mit Erdalkalimetallionen gebildeten Komplexe beschrieben. Für die mit Serin-N,N-diessigsäure gebildeten Komplexe mit Erdalkalimetallionen wird dabei festgestellt, daß ihre Stabilität geringer als erwartet ist, da man die Stabilitätswerte der Nitrilotriessigsäure glaubte erreichen zu können.

Die Anwendbarkeit dieser Verbindungen als Hilfskomplexbildner wurde untersucht, indem sie zu Zink-, Eisen-III-, Kupfer- und Nickellösungen, jeweils bei einem pH-Wert von 13,5, sowie zu Aluminiumlösungen bei einem pH-Wert von 7, zugegeben wurden. Für Serin-N,N-diessigsäure wird dabei festgestellt, daß diese Zink- und Kupferionen bei einem Molverhältnis Metallion : Komplexbildner von 1 : 2 in Lösung hält, wobei überschüssige Metallionen ausgeschieden werden. Als zusammenfassendes Ergebnis wird festgestellt, daß man die untersuchten Verbindungen als Maßlösungen, d.h. für die Analytik von Erdalkalilösungen, nur mit sehr begrenzten Möglichkeiten benutzen kann und daß sie eventuell als Hilfskomplexbildner für Schwermetallionen in Betracht kommen können.

Das aus diesen Ergebnissen hervorgehende mangelnde Komplexbindungsvermögen vermag dem Fachmann keine Anregung dafür zu geben, 2-Hydroxy-3-aminopropionsäure-N,N-diessigsäure (Isoserin-N,N-diessigsäure) und ihre Derivate herzustellen und als Komplexbildner zu verwenden.

Aufgabe der vorliegenden Erfindung ist es, neue Komplexbildner für Erdalkali- und Schwermetallionen für die verschiedensten technischen Gebiete, insbesondere für Waschmittel, zur Verfügung zu stellen, die neben guten komplexbildenden Eigenschaften ökologisch unbedenklich, möglichst keinen Phosphor enthalten und biologisch gut abbaubar sind. Für diese neuen Komplexbildner sollen auch technisch vorteilhafte Herstellverfahren entwickelt werden.

Es wurde nun gefunden, daß 2-Hydroxy-3-aminopropionsäure-N,N-diessigsäure und ihre Derivate der Formel I

$$(Y{-}CH_2)_2NCH_2\underset{\displaystyle OH}{\underset{|}{CH}}{-}(COX) \qquad (I)$$

in der Y für den Rest $-COOH$, der gegebenenfalls in Form eines Alkalimetall-, Ammonium- oder substituierten Ammoniumsalzes vorliegt, für einen Rest $-COOR^1$, in dem $R^1$ einen Alkylrest mit 1 bis 4 C-Atomen bedeutet, oder für den Rest $-CN$ steht und
X eine Hydroxylgruppe, wobei die dadurch vorliegende Carbonsäuregruppe gegebenenfalls in Form eines Alkalimetall-, Ammonium- oder substituierten Ammoniumsalzes vorliegt, einen Rest $-OR^2$, in dem $R^2$ für einen Alkylrest mit 1 bis 4 C-Atomen steht, oder einen Rest $-NR^3R^4$, in dem $R^3$ und $R^4$ gleich oder verschiedenen sind und für ein Wasserstoffatom oder einen Alkylrest mit 1 bis 4 C-Atomen stehen,
bedeutet, in der Form der freien Säure oder insbesondere der Natrium-, Kalium, Ammonium- oder organischen Aminsalze hervorragende neue Komplexbildner insbesondere für Calcium-, Magnesium- sowie Eisen-, Kupfer-, Nickel- und Manganionen darstellen, während die Säurederivate, wie Ester, Amide und Nitrile, bevorzugt Zwischenprodukte für die Herstellung der Säure und ihre Salze sind.

Ein bevorzugter Gegenstand der vorliegenden Erfindung sind daher neue Komplexbildner für Schwermetall- und Erdalkaliionen der Formel I, in der Y den Rest $-COOH$ und X $-OH$ bedeuten, wobei die vorliegenden Carbonsäuregruppe gegebenenfalls in Form der Alkalimetall-, Ammonium- oder substituierten Ammoniumsalze vorliegen.

Im einzelnen sind zu nennen die freie 2-Hydroxy-3-amino-propionsäure-N,N-diessigsäure und die Natrium- Kalium- und Ammoniumsalze, insbesondere das Trinatrium-, Trikalium- und Triammoniumsalz sowie organische Triaminsalze mit einem tertiären Stickstoffatom.

Als den organischen Aminsalzen zugrundeliegende Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin oder Tripropanolamin, in Betracht.

Die Verbindungen der Formel I können hergestellt werden, indem man eine Verbindung der Formel II

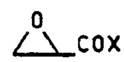

(II)

in der X die für Formel I angegebenen Bedeutungen aufweist, mit einer Verbindung der Formel III

$$HN(CH_2-Y)_2 \quad \text{(III)}$$

in der Y die für Formel I genannten Bedeutungen aufweist, in Wasser, einem organischen Lösungsmittel oder ihren Mischungen bei Temperaturen von 10 bis 80°C umsetzt und gegebenenfalls im Falle des Vorliegens von Amid-, Ester- oder Nitrilgruppen diese in Gegenwart von einer Säure oder Base hydrolysiert und gegebenenfalls die freie Säure oder ein Salz gemäß Formel I isoliert.

Die verwendeten Ausgangsverbindungen der Formeln II und III sind bekannt oder können ohne weiteres nach bekannten Verfahren hergestellt werden.

Bevorzugte Ausgangsverbindungen der Formel II sind Glycidylsäure, gegebenenfalls in Form des Natrium- Kalium- oder Ammoniumsalzes, das Glycidylsäureamid oder ein Glycidylsäureester mit einem einwertigen Alkohol mit 1 bis 4 C-Atomen als Esteralkohol.

Für Ausgangsverbindungen der Formel III stehen vorzugsweise die Iminodiessigsäure, gegebenenfalls in Form der Mono- oder Di-natrium-, -kalium- oder -ammoniumsalze, das Iminodiacetonitril, der Iminodiessigsäuremethyl oder -ethylester.

Die Ausgangsverbindungen der Formel II und III werden vorzugsweise in äquimolaren Mengen umgesetzt.

Als Lösemittel kommen vorzugsweise Wasser zum Einsatz, oder mit Wasser mischbare organische Lösemittel, wie Methanol, Ethanol, n-Propanol, iso-Propanol, tertiär-Butanol, Dioxan oder Tetrahydrofuran. Es können auch Mischungen dieser organischen Lösungsmittel untereinander oder ihre Mischungen mit Wasser verwendet werden. Bei wäßrigen Mischungen wird zweckmäßigerweise zum Gewicht des Wassers 10 bis 70 Gew.% organisches Lösungsmittel gegeben. Gegebenenfalls ist auch eine Umsetzung ohne Lösungsmittel möglich, wenn beispielsweise von einem flüssigen Ester, wie einem Glycidylester oder einem Iminodiessigsäurediester, ausgegangen wird.

Die Konzentration der Ausgangsmaterialien im jeweiligen Lösungsmittel beträgt vorteilhaft 10-80 Gew.%, vorzugsweise 20-70 Gew.%.

Die Umsetzung erfolgt zweckmäßig bei Temperaturen von 10 bis 80°C, vorzugsweise von 40 bis 70°C.

Die Umsetzung erfolgt zweckmäßigerweise bei pH-Werten von 4 bis 10, bevorzugt 6 bis 9. Sie ist bei Normaldruck, aber auch erhöhtem Druck möglich.

Im Falle des Vorliegens von Amid-, Ester- oder Nitrilgruppen wird eine Hydrolyse oder Verseifung zur Tricarbonsäure in an sich üblicher Weise in einem wäßrigen Reaktionsgemisch gegebenenfalls nach Zusatz von Wasser in Gegenwart von Alkalien, wie Natrium- oder Kaliumhydroxid, oder von Säure, wie Schwefelsäure oder Salzsäure, durchgeführt.

Diese Hydrolyse wird zweckmäßigerweise bei Temperaturen von 20 bis 110°C, vorzugsweise 40 bis 100°C, durchgeführt bei gegebenenfalls geringem Überschuß an Base oder Säure.

Entsprechend den Reaktionsbedingungen wird bevorzugt die 2-Hydroxy-3-amino-propionsäure-N,N-diessigsäure oder eine Alkalisalz erhalten. Anschließend können ohne weiteres Salze mit einem anderen Kation hergestellt werden.

Falls erforderlich, kann auch umgekehrt aus der erhaltenen Säure in üblicher Weise ein Säurederivat hergestellt werden.

Die Verbindungen der Formel I können ohne Schwierigkeiten in reiner Form isoliert werden. Für der freie Säure und die Salze bieten sich insbesondere Sprüh- oder Gefriertrocknung, Kristallisation oder Fällung an. Es kann vorteilhaft sein, die angefallene Lösung direkt einer technischen Verwendung zuzuführen.

Nach weiteren Verfahren können die Verbindungen der Formel I hergestellt werden, indem man 1 Mol 2-Hydroxy-3-aminopropionsäure gegebenenfalls in Form eines Alkalisalzes, eines Alkylesters mit 1 bis 4 C-Atomen im Alkyl oder des Säureamids, gegebenenfalls am Amidstickstoffatom durch ein oder zwei Alkylreste mit 1 bis 4 C-Atomen substituiert, in Wasser, einem organischen Lösungsmittel oder ihren Mischungen bei Temperaturen von 0 bis 100°C mit 2,0 bis 2,6 Mol einer Monohalogenessigsäure, einem Monohalogenessigsäurealkylester mit 1 bis 4 C-Atomen im Alkyl oder Monohalogenacetonitril in alkalischem Medium oder in Gegenwart eines Säurefängers umsetzt und gegebenenfalls die vorliegenden Amid-, Ester- und Nitrilgruppen in Gegenwart von einer Säure oder Base hydrolysiert und gegebenenfalls die freie Säure oder ein Salz gemäß Formel I isoliert.

Nach einem weiteren Verfahren zur Herstellung von Verbindungen der Formel I, in der Y die für Formel I angegebenen Bedeutungen außer dem Rest $-COOR^1$ und X die für Formel I angegebenen Bedeutungen aufweist, werden 1 Mol 2-Hydroxy-3-aminopropionsäure gegebenenfalls in Form eines Alkalisalzes, eines Alkylesters mit 1 bis 4 C-Atomen im Alkyl oder des Säureamids, gegebenenfalls am Amidstickstoffatom durch ein oder zwei Alkylreste mit 1 bis 4 C-Atomen substituiert, in Wasser, einem organischen Lösungsmittel oder ihren Mischungen mit 2,0 bis 2,6 Mol Formaldehyd und 2 bis 2,3 Mol flüssiger Blausäure bei Temperaturen von 0 bis 45°C oder 2 bis 2,3 Mol Alkalicyanid bei Temperaturen von 40 bis 100°C umgesetzt und gegebenenfalls die vorliegenden Amid-, Ester- und Nitrilgruppen in Gegenwart von einer Säure oder Base hydrolysiert und gegebenenfalls die freie Säure oder ein Salz gemäß Formel I isoliert.

Als Alkalisalze der 2-Hydroxy-3-aminopropionsäure werden bei diesen Verfahren das Natrium- oder das Kaliumsalz bevorzugt.

Die Herstellung der 2-hydroxy-3-amino-propionsäure als Ausgangsverbindung erfolgt zweckmäßig durch Umsetzung einer Glycidylverbindung der Formel II, bevorzugt von Glycidylsäure, ihrem Natrium- oder Kaliumsalz, Glycidylamid oder einem Ester in wässriger, organischer oder wäßrig-organischer Lösung, wie oben angegeben, mit 1 bis 2 Mol, bevorzugt 1 bis 1,4 Mol, Ammoniak pro Mol Glycidylverbindung der Formel II bei Temperaturen von 10 bis 80°C und pH-Werten von 6 bis 9.

Bei der Umsetzung mit einer Monohalogenessigsäureverbindung sind Chloressigsäure oder Bromessigsäure bevorzugt und Temperaturen von 40 bis 80°C. Als alkalisches Medium kommen die oben genannten Lösungsmittel oder Gemische in Betracht, wobei bevorzugt ist eine wäßrige Lösung mit einem pH-Wert von 7,5 bis 12, zweckmäßig eingestellt mit NaOH oder KOH, so daß die Salze vorliegen, oder die Gegenwart von bevorzugt eines tertiären Amins als Säurefänger. Zweckmäßige tertiäre Amine sind Trialkylamine mit 1 bis 4 C-Atomen im Alkyl, wie Trimethyl-, Triethyl-, Tri-n-propyl-, Triisopropyl-, Tri-n-butyl- und Triisobutylamin sowie cyclische tertiäre Amine, wie 1,4-Diazabicyclo[2,2,2]octan.

Bei einer zweckmäßigen Reaktionsführung nach Art der Strecker'schen Synthese in an sich üblicher Weise, vgl. Houben-Weyl, Bd. 11/2, S. 408-412 (1958), Thieme-Verlag Stuttgart, wird das Natrium- oder Kaliumsalz der 2-Hydroxy-3-aminopropionsäure in einem der oben genannten Lösungsmittel oder Lösungsmittelgemisches, wobei eine wäßrige Lösung bevorzugt wird, mit dem Formaldehyd in Form seiner wäßrigen, ca. 30 gew.% igen Lösungen und der flüssigen Blausäure bevorzugt bei 15 bis 25°C umgesetzt. Die Umsetzung mit Alkalicyanid, Natrium- oder Kaliumcyanid, anstelle flüssiger Blausäure erfolgt bevorzugt bei 70 bis 100°C.

Für die Umsetzung mit freier Blausäure kommen zweckmäßigerweise pH-Wert-Bereiche von 0 bis 11, vorzugsweise 2 bis 9, in Betracht.

Die gegebenenfalls erforderliche Hydrolyse vorhandener Ester-, Amid- und Nitrilgruppen, Isolierung der Säure oder Salze erfolgt wie oben angegeben.

Nach einem weiteren Verfahren zur Herstellung von Verbindungen der allgemeinen Formel I werden 1 Mol α-Chloracrylsäure, gegebenenfalls in form eines Alkalisalzes, insbesondere Na-Salzes, oder eines Alkylesters mit 1 bis 4 C-Atomen, in Wasser, einem organischen Lösungsmittel oder ihren Mischungen mit 1,0 bis 1,2 Mol

einer Verbindung der allgemeinen Formel III bei Temperaturen von 20 bis 150°C umgesetzt und die gegebenenfalls vorliegenden Ester und/oder Nitrilgruppen sowie das noch enthaltende Chlor in üblicher Weise in Gegenwart einer Base hydrolysiert und durch OH-ersetzt und die freie Säure oder ein Salz gemäß Formel I isoliert.

Die durch die Erfindung erstmals hergestellte 2-Hydroxy-3-amino-propionsäure-N,N-diessigsäure und ihre Salze sind in hervorragender Weise geeignet, Erdalkali- und Schwermetallionen zu komplexieren. Aufgrund dieser Fähigkeit weisen sie eine Vielzahl von technischen Anwendungsmöglichkeiten auf. Da es sich um biologisch sehr gut abbaubare Verbindungen handelt, können sie in großen Mengen überall dort eingesetzt werden, wo die Abwässer geklärt werden müssen und auch phosphorhaltige Verbindungen vermieden werden sollen.

In Wasch- und Reinigungsmitteln können die erfindungsgemäßen Komplexbildner eingesetzt werden, um den Gehalt an freien Schwermetallionen in den Waschmitteln selbst und in den Waschlösungen zu kontrollieren. Die Einsatzmenge als Komplexbildner beträgt zweckmäßigerweise 0,1 bis 2%, bezogen auf das Gesamtgewicht der Waschmittelbestandteile.

Ihre vorteilhafte Wirkung liegt auch in einer Bleichmittelstabilisierung, beispielsweise für Natriumperborat, in Waschmitteln und bei der Bleiche von Textilien, Zellstoff oder Papierrohstoff. Spuren von Schwermetallen, wie Eisen, Kupfer und Mangan, kommen im Waschpulver selbst, im Wasser und im Textilgut vor und katalysieren die Zersetzung des Natriumperborates. Die erfindungsgemäßen Komplexbildner binden diese Metallionen und verhindern die unerwünschte Zersetzung des Bleichsystems während der Lagerung und in der Waschflotte. Dadurch erhöht sich die Effizienz des Bleichsystems und Faserschädigungen werden zurückgedrängt.

Zusätzlich werden Enzyme, opt. Aufheller und Duftstoffe vor schwermetallkatalysierter oxidativer Zersetzung geschützt.

In flüssigen Waschmittelformulierungen können die neuen Komplexbildner als sog. Konservierungsmittel, zweckmäßig in einer Menge von 0,05 bis 1 Gew.%, bezogen auf das Gesamtgewicht der Waschmittelformulierung, eingesetzt werden.

In Seifen verhindern die neuen Komplexbildner beispielsweise metallkatalysierte oxidative Zersetzungen.

Weiterhin dienen sie in hervorragender Weise in Wasch- und Reinigungsmitteln als Gerüststoff (Builder), um Ausfällungen und Inkrustationen auf dem Gewebe zu verhindern.

Sie können in vorteilhafter Weise überall dort eingesetzt werden, wo bei technischen Verfahren Ausfällungen von Ca-, Mg- und Schwermetallsalzen stören und verhindert werden sollen. Beispielsweise zur Verhinderung von Ablagerungen und Verkrustungen in Kesseln, Rohrleitungen, an Sprühdüsen oder allgemein an glatten Oberflächen.

Sie können zur Stabilisierung von Phosphaten in alkalischen Entfettungsbädern und Verhinderung der Ausfällung von Kalkseifen dienen und verhindern dadurch das "Anlaufen" von Nichteisenoberflächen und verlängern die Standzeiten von alkalischen Reinigerbädern.

Sie können als Komplexbildner in alkalischen Entrostungs- und Entzunderungsbädern verwendet werden sowie in galvanischen Bädern anstelle von Cyaniden, um Verunreinigungen zu maskieren.

Die Kühlwasserbehandlung mit den neuen Komplexbildern verhindert Ablagerungen bzw. löst bereits vorhandene wieder auf. Von Vorteil ist die Anwendung in alkalischem Medium und damit die Beseitigung von Korrosionsproblemen.

Bei der Polymerisation von Kautschuk können sie zur Herstellung der dabei verwendeten Redoxkatalysatoren verwendet werden. Sie verhindern zusätzlich das Ausfallen von Eisenhydroxid im alkalischen Polymerisationsmilieu.

In der photographischen Industrie können die neuen Komplexbildner in Entwickler/Fixierbädern, die mit hartem Wasser angesetzt werden, verwendet werden, um die Ausfällung schwerlöslicher Ca- und Mg-salze zu verhindern. Die Ausfällungen führen zur Grauschleiern auf Filmen und Bildern sowie Ablagerungen in den Tanks, die somit vorteilhaft vermieden werden können. Eisen-III-Komplexbilnerlösungen können vorteilhaft in Bleichfixierbädern eingesetzt werden, wo sie die aus ökologischen Gründen bedenklichen Hexacyanoferratlösungen ersetzen können.

In der Textilindustrie können sie zur Entfernung von Schwermetallspuren während des Herstellungs- bzw. Färbeprozesses von natürlichen und synthetischen Fasern dienen. Dadurch werden viele Störungen verhindert : Schmutzflecken und Streifen auf dem Textilgut, Verlust des Glanzes, schlechte Benetzbarkeit, unegale Färbungen und Farbfehler.

In der Papierindustrie können sie zur Eliminierung von Schwermetall/Eisenionen verwendet werden. Die Ablagerung von Eisen auf Papier führt zu "heißen Flecken", an denen die oxidative, katalytische Zerstörung der Zellulose beginnt.

Als verschiedene Anwendungen kommen beispielsweise Anwendungen in Pharmazeutika, Kosmetika und

Nahrungsmitteln in Betracht, um die metallkatalysierte Oxidation von olefinischen Doppelbindungen und damit das Ranzigwerden der Erzeugnisse zu verhindern.

In der Pflanzenernährung werden zur Behebung von Schwermetalldefiziten Cu, Fe, Mn, Zn-Komplexe verwendet. Die Schwermetalle werden als Chelate zugegeben, um die Ausfällung als biologisch inaktive, unlösliche Salze zu verhindern.

Weitere Anwendungsgebiete für die neuen Komplexbildner sind die Rauchgaswäsche, und zwar die Entfernung von $NO_x$ aus Rauchgasen, die $H_2S$-Oxidation, die Metallextraktion, sowie Anwendungen als Katalysatoren für org. Synthesen (z.B. Luftoxidation von Paraffinen, Hydroformylierung von Olefinen zu Alkoholen).

Die erfindungsgemäßen Komplexbildner für Erdalkali- und Schwermetallionen werden als Komplexbildner allgemein und ganz besonders hervorzuheben in Wasch- und Reinigungsmitteln sowie Spül- und Waschhilfsmitteln, insbesondere als Komplexbildner für Schwermetall- und/oder Erdalkaliionen, als Bleichmittelstabilisatoren sowie als Gerüststoffe (Builder) verwendet.

Gegenstand der Erfindung sind demnach auch die entsprechenden Verwendungszwecke und Wasch- und Reinigungsmittel, die diese Verbindungen neben den üblichen, dem Fachmann bekannten Bestandteilen enthalten.

Die erfindungsgemäß zu verwendenden Verbindungen werden in Wasch- und Reinigungsformulierungen im allgemeinen in eine Menge von 0,01 bis 20 Gew.%, bevorzugt 0,05 bis 10 Gew.%, bezogen auf das Gesamtgewicht der Waschmittelformulierung, eingesetzt.

Bei einer Verwendung bevorzugt als Gerüststoff sind Mengen von 1 bis 10 Gew.%, bei einer Verwendung bevorzugt als Bleichmittelstabilisator für Perborate sind Mengen von 0,05 bis 1 Gew.% besonders bevorzugt. Bei einer Verwendung insbesondere als Komplexbildner in Waschmitteln sind Mengen von 0,1 bis 2 Gew.% bevorzugt.

Wasch- und Reinigungsmittelformulierungen, die, bezogen auf das Gesamtgewicht, 0,01 bis 20, bevorzugt 0,05 bis 10 Gew.%, erfindungsgemäß zu verwendende Verbindung enthalten, enthalten in der Regel als zusätzliche Bestandteile, bezogen auf das Gesamtgewicht, 6 bis 25 Gew.% Tenside, 15 bis 50 Gew.% Builder und gegebenenfalls Co-Builder, 5 bis 35 Gew.% Bleichmittel und gegebenenfalls Bleichmittelaktivatoren, 3 bis 30 Gew.% Hilfsstoffe, wie Enzyme, Schaumregulatoren, Korrosionsinhibitoren, optische Aufheller, Duftstoffe, Farbstoffe, oder Formulierhilfsmittel, wie z.B. Natriumsulfat.

Die erfindungsgemäßen Verbindungen können in ihrer Eigenschaft als Komplexbildner, Gerüststoffe und Bleichmittelstabilisatoren auch verwendet werden in Wasch- und Reinigungsformulierungen zusammen mit anderen Mitteln des Standes der Technik, wobei die allgemeinen Eigenschaften im Hinblick auf Sequestrierung, Inkrustationsinhibierung, Vergrauungsinhibierung, Primärwaschwirkung und Bleichwirkung unter Umständen deutlich verbessert werden können.

Übliche, dem Fachmann bekannte Bestandteile von Waschmittelformulierungen, bezogen auf die o.a. Rahmenvorschrift, seien im folgenden beispielsweise aufgezählt:

Geeignete Tenside sind solche, die im Molekül wenigstens einen hydrophoben organischen Rest und eine wasserlöslich machende anionische, zwitterionische oder nichtionische Gruppe enthalten. Bei dem hydrophoben Rest handelt es sich meist um einen aliphatischen Kohlenwasserstoffrest mit 8 bis 26, vorzugsweise 10 bis 22 und insbesondere 12 bis 18 C-Atomen, oder um einen alkylaromatischen Rest mit 6 bis 18, vorzugsweise 8 bis 16 aliphatischen C-Atomen.

Geeignete synthetische anionische Tenside sind insbesondere solche vom Typ der Sulfonate, Sulfate oder der synthetischen Carboxylate.

Als Tenside vom Sulfonattyp kommen Alkylbenzolsulfonate mit 4 bis 15 C-Atomen im Alkyl, Gemische aus Alken- und Hydroxyalkansulfonaten sowie Disulfonaten, wie man sie beispielsweise aus Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Weiter eignen sich Alkansulfonate, die aus Alkanen durch Sulfochlorierung oder Sulfoxydation und anschließende Hydrolyse bzw. Neutralisation bzw. durch Bisulfitaddition an Olefine erhältlich sind. Weitere brauchbare Tenside vom Sulfonattyp sind die Ester von α-Sulfofettsäuren, z.B. die α-Sulfonsäuren aus hydrierten Methyl- oder Ethylestern der Kokos-, Palmkern- oder Talgfettsäure.

Geeignete Tenside vom Sulfattyp sind die Schwefelsäuremonoester primärer Alkohole, z.B. aus Kokosfettalkoholen, Talgfettalkoholen oder Oleylalkohol, und solche von sekundären Alkoholen. Weiterhin eignen sich sulfatierte Fettsäurealkanolamine, Fettsäuremonoglyceride oder Umsetzungsprodukte von 1 bis 4 Mol Ethylenoxid mit primären oder sekundären Fettalkoholen oder Alkylphenolen.

Weitere geeignete anionische Tenside sind die Fettsäureester bzw. -amide von Hydroxy- oder Aminocarbonsäuren bzw. -sulfonsäuren, wie z.B. die Fettsäuresarcoside, -glykolate, -lactate, -tauride oder -isothionate.

Die anionischen Tenside können in Form ihrer Natrium-, Kalium- und Ammoniumsalze sowie als lösliche Salze organischer Basen, wie Mono- Di- oder Triethanolamin, vorliegen. Die üblichen Seifen, d.h. Salze der

natürlichen Fettsäuren, sollen nicht unerwähnt bleiben.

Als nichtionische Tenside (Nonionics) sind z.B. Anlagerungsprodukte von 3 bis 40, vorzugsweise 4 bis 20 Mol Ethylenoxid an 1 Mol Fettalkohol, Alkylphenol, Fettsäure, Fettamin, Fettsäureamid oder Alkansulfonamid verwendbar. Besonders wichtig sind die Anlagerungsprodukte von 5 bis 16 Mol Ethylenoxid an Kokos- oder Talgfettalkohole, an Oleylalkohol oder an synthetische Alkohole mit 8 bis 18, vorzugsweise 12 bis 18 C-Atomen, sowie an Mono- oder Dialkylphenole mit 6 bis 14 C-Atomen in den Alkylresten. Neben diesen wasserlöslichen Nonionics sind aber auch nicht bzw. nicht vollständig wasserlösliche Polyglykolether mit 1 bis 4 Ethylenglykoletherresten im Molekül von Interesse, insbesondere wenn sie zusammen mit wasserlöslichen nichtionischen oder anionischen Tensiden eingesetzt werden.

Weiterhin sind als nichtionische Tenside die wasserlöslichen, 20 bis 250 Ethylenglykolethergruppen und 10 bis 100 Propylenglykolethergruppen enthaltenden Anlagerungsprodukte von Ethylenoxid an Polypropylenglykolether, Alkylendiaminopolypropylenglykol und Alkylpolypropylenglykole mit 1 bis 10 C-Atomen in der Alkylkette brauchbar, in denen die Polypropylenglykoletherkette als hydrophober Rest fungiert.

Auch nichtionische Tenside vom Typ der Aminoxide oder Sulfoxide sind verwendbar.

Das Schaumvermögen der Tenside läßt sich durch Kombination geeigneter Tensidtypen steigern oder verringern. Eine Verringerung läßt sich ebenfalls durch Zusätze von nichttensidartigen organischen Substanzen erreichen.

Als Buildersubstanzen sind beispielsweise geeignet : Waschalkalien, wie Natriumcarbonat und Natriumsilicat, oder Komplexbildner, wie Phosphate, oder Ionenaustauscher, wie Zeolithe, sowie deren Mischungen. Diese Gerüst- und Aufbaustoffe haben die Aufgabe, die teils aus Wasser, teils aus Schmutz oder dem Textilgut stammenden Härteionen zu eliminieren und die Tensidwirkung zu unterstützen. Neben den o.g. Buildersubstanzen können weiter im Builder sogenannte Co-builder enthalten sein. Die Co-builder haben in modernen Waschmitteln die Aufgabe, einige Eigenschaften der Phosphate zu übernehmen, wie z.B. Sequestrierwirkung, Schmutztragevermögen, Primär- und Sekundärwaschwirkung.

Im Builder können z.B. wasserunlösliche Silicate, wie sie beispielsweise in der DE-OS 24 12 837 beschrieben werden, und/oder Phosphate vorhanden sein. Aus der Gruppe der Phosphate können Pyrophosphat, Triphosphat, höhere Polyphosphate und Metaphosphate verwendet werden. Auch phosphorhaltige organische Komplexbildner, wie Alkanpolyphosphonsäuren, Amino- und Hydroxyalkanpolyphosphonsäuren und Phosphonocarbonsäuren kommen als weitere Waschmittelinhaltsstoffe in Betracht. Beispiele solcher Waschmitteladditive sind z.B. die folgenden Verbindungen : Methandiphosphonsäure, Propan-1,2,3-triphosphonsäure, Butan-1,2,3,4-tetraphosphonsäure, Polyvinylphosphonsäure, 1-Aminoethan-1,1-diphosphonsäure, 1-Amino-1-phenyl-1,1-diphosphonsäure, Aminotrismethylen-triphosphonsäure, Methylamino- oder Ethylaminobismethylendiphosphonsäure, Ethylendiaminotetramethylentetraphosphonsäure, Diethylentriaminopentamethylenpentaphosphonsäure, 1-Hydroxethan-1,1-diphosphonsäure, Phosphonoessig- und Phosphonopropionsäure, Mischpolymerisate aus Vinylphosphonsäure und Acryl- und/oder Maleinsäure, sowie deren teil- oder vollneutralisierten Salze.

Weitere organische Verbindungen, die als Komplexierungsmittel für Calcium wirken und in Waschmittelformulierungen enthalten sein können, sind Polycarbonsäuren, Hydroxycarbonsäuren und Aminocarbonsäuren, die meist in Form ihrer wasserlöslichen Salze eingesetzt werden.

Beispiele für Polycarbonsäuren sind Dicarbonsäuren der allgemeinen Formel $HOOC-(CH_2)_m-COOH$ mit $m=0-8$, außerdem Maleinsäure, Methylenmalonsäure, Citraconsäure, Mesaconsäure, Itaconsäure, nicht cyclische Polycarbonsäuren mit wenigstens 3 Carboxylgruppen im Molekül, wie z.B. Tricarballylsäure, Aconitsäure, Ethylentetracarbonsäure, 1,1,3-Propan-tetracarbonsäure, 1,1,3,3,5,5-Pentan-hexacarbonsäure, Hexanhexacarbonsäure, cyclische Di- oder Polycarbonsäuren, wie z.B. Cyclopentan-tetracarbonsäure, Cyclohexan-hexacarbonsäure, Tetrahydrofurantetracarbonsäure, Phthalsäure, Terephthalsäure, Benzoltri-, -tetra- oder -pentacarbonsäure sowie Mellithsäure.

Beispiele für Hydroxymono- oder -polycarbonsäuren sind Glykolsäure, Milchsäure, Äpfelsäure, Tartronsäure, Methyltartronsäure, Gluconsäure, Glycerinsäure, Citronensäure, Weinsäure, Salicylsäure.

Beispiele für Aminocarbonsäuren sind Glycin, Glyclglycin, Alanin, Asparagin, Glutaminsäure, Aminobenzoesäure, Iminodi- oder -triessigsäure, Hydroxyethyliminodiessigsäure, Ethylendiaminotetraessigsäure, Hydroxyethyl-ethylendiamin-triessigsäure, Diethylentriamin-pentaessigsäure sowie höhere Homologe, die durch Polymerisation eines N-Aziridylcarbonsäurederivates, z.B. der Essigsäure, Bernsteinsäure, Tricarballylsäure, und anschließende Verseifung, oder durch Kondensation von Polyaminen mit einem Molekulargewicht von 500 bis 10000 mit chloressigsauren oder bromessigsauren Salzen hergestellt werden können.

Als Co-Buildersubstanzen werden bevorzugt polymere Carbonsäuren verwendet. Zu diesen polymeren Carbonsäuren sind die Carboxymethylether der Zucker, der Stärke und der Cellulose zu zählen.

Unter den polymeren Carbonsäuren spielen z.B. die Polymerisate der Acrylsäure, Maleinsäure, Itaconsäure, Mesaconsäure, Aconitsäure, Methylenmalonsäure, Citraconsäure und dgl., die Copolymerisate der

oben genannten Carbonsäuren untereinander, wie z.B. ein Copolymerisat aus Acrylsäure und Maleinsäure im Verhältnis 70 : 30 und vom Molgewicht 70000, oder mit ethylenisch ungesättigten Verbindungen, wie Ethylen, Propylen, Isobutylen, Vinylalkohol, Vinylmethylether, Furan, Acrolein, Vinylacetat, Acrylamid, Acrylnitril, Methacrylsäure, Crotonsäure etc. wie z.B die 1 : 1-Mischpolymerisate aus Maleinsäureanhydrid und Methylvinylether vom Molgewicht 70000 oder die Mischpolymerisate von Maleinsäureanhydrid und Ethylen bzw. Propylen bzw. Furan eine besondere Rolle.

In den Co-Buildern können weiterhin Schmutzträger enthalten sein, die den von der Faser abgelösten Schmutz in der Flotte suspendiert halten und so das Vergrauen inhibieren. Hierzu sind wasserlösliche Kolloide meist organischer Natur geeignet, wie beispielsweise die wasserlöslichen Salze polymerer Carbonsäuren, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche saure Gruppen enthaltende Polyamide sind für diesen Zweck geeignet. Weiterhin lassen sich lösliche Stärkepräparate und andere als die oben genannten Stärkeprodukte verwenden, wie z.B. abgebaute Stärke, Aldehydstärken usw. Auch Polyvinylpyrrolidon ist brauchbar.

Bleichmittel sind insbesondere Wasserstoffperoxid und Derivate oder Aktivchlor-liefernde Verbindungen. Unter den als Bleichmittel dienenden, in Wasser $H_2O_2$ liefernden Verbindungen haben Natriumperborat-Hydrate, wie $NaBO_2 \cdot H_2O_2 \cdot 3H_2O$ und $NaBO_2 \cdot H_2O_2$, besondere Bedeutung. Es sind aber auch andere $H_2O_2$ liefernde Borate brauchbar. Diese Verbindungen können teilweise oder vollständig durch andere Aktivsauerstoffträger, insbesondere durch Peroxyhydrate, wie Peroxycarbonate, Peroxyphosphonate, Citratperhydrate, Harnstoff $-H_2O_2-$ oder Melamin $-H_2O_2-$ Verbindungen sowie durch $H_2O_2$ liefernde persaure Salze, wie z.B. Caroate, Perbenzoate oder Peroxyphthalate, ersetzt werden.

Es können neben den erfindungsgemäßen übliche wasserlösliche und/oder wasserunlösliche Stabilisatoren für die Peroxyverbindungen zusammen mit diesen in Mengen von 0,25 bis 10 Gew.%, bezogen auf die Peroxiverbindung, eingearbeitet werden. Als wasserunlösliche Stabilisatoren eignen sich die meist durch Fällung aus wäßrigen Lösungen erhaltenen Magnesiumsilikate $MgO : SiO_2$ der Zusammensetzung 4 : 1 bis 1 : 4, vorzugsweise 2 : 1 bis 1 : 2 und insbesondere 1 : 1. An deren Stelle sind auch andere Erdalkalimetalle entsprechender Zusammensetzung brauchbar.

Um beim Waschen bereits bei Temperaturen unterhalb 80°C, insbesondere im Bereich von 60 bis 40°C, eine befriedigende Bleichwirkung zu erreichen, werden zweckmäßigerweise Bleichaktivatoren in die Waschmittel eingearbeitet, vorteilhaft in einer Menge von, bezogen auf die $H_2O_2$-liefernde Verbindung, von 5 bis 30 Gew.%.

Als Aktivatoren für in Wasser $H_2O_2$ liefernde Perverbindungen dienen bestimmte, mit $H_2O_2$ organische Persäuren bildende N-Acyl, O-Acyl-Verbindungen, insbesondere Acetyl-, Propionyl- oder Benzoylverbindungen, sowie Kohlensäure- bzw. Pyrokohlensäureester. Brauchbare Verbindungen sind unter anderen :
N-diacylierte und N,N'-tetraacylierte Amine, wie z.B.
N,N,N',N'-Tetraacetyl-methylendiamin oder -ethylendiamin,
N,N-Diacetylanilin und N,N-Diacetyl-p-toluidin bzw. 1,3-diacylierte Hydantoine,
Alkyl-N-sulfonyl-carbonamide,
N-acylierte cyclische Hydrazide, acylierte Triazole oder Urazole, wie z.B. das
Monoacetylmaleinsäurehydrazid,
O,N,N-trisubstituierte Hydroxylamine, wie z.B.
O-Benzoyl-N,N-succinyl-hydroxylamin,
O-Acetyl-N,N-succinyl-hydroxylamin,
O-p-Methoxybenzoyl-N,N-succinylhydroxylamin,
O-p-Nitrobenzoyl-N,N-succinylhydroxylamin und
O,N,N-Triacetyl-hydroxylamin,
Carbonsäureanhydride, z.B.
Benzoesäureanhydrid,
m-Chlorbenzoesäureanhydrid,
Phthalsäureanhydrid, 4-Chlorphthalsäureanhydrid,
Zuckerester, wie z.B. Glucosepentaacetat,
Imidazolidinderivate, wie
1,3-Diformyl-4,5-diacetoxyimidazolidin,
1,3-Diacetyl-4,5-diacetoxy-imidazolidin,
1,3-Diacetyl-4,5-di-propionyloxy-imidazolidin,
acylierte Glykolurile, wie z.B.
Tetrapropionylglykoluril oder
Diacetyl-dibenzoylglykoluril,

dialkylierte 2,5-Diketopiperazine, wie z.B.
1,4-Diacetyl-2,5-diketopiperazin,
1,4-Dipropionyl-2,5-diketopiperazin,
1,4-Dipropionyl-3,6-dimethyl-2,5-diketopiperazin,
Acetylierungs- bzw. Benzoylierungsprodukte von Propylendiharnstoff bzw.
2,2-Dimethyl-propylendiharnstoff,
das Natriumsalz der p-(Ethoxycarbonyloxy)-benzoesäure und der
p-(Propoxycarbonyloxy)-benzolsulfonsäure sowie die Natriumsalze von alkylierten oder acylierten Phenolsulfonsäureestern, wie
p-Acetoxy-benzolsulfonsäure, 2-Acetoxy-5-nonyl-benzolsulfonsäure,
2-Acetoxy-5-propylbenzolsulfonsäure oder der
Isononanoyloxiphenylsulfonsäure.

Als Bleichmittel können auch Aktivchlorverbindungen anorganischer oder organischer Natur eingesetzt werden. Zu den anorganischen Aktivchlorverbindungen gehören Alkalihypochlorite, die insbesondere in Form ihrer Mischsalze bzw. Anlagerungsverbindungen an Orthophosphate oder kondensierte Phosphate, wie beispielsweise an Pyro- und Polyphosphate oder an Alkalisilikate, verwendet werden können. Enthalten die Wasch- und Waschhilfsmittel Monopersulfate und Chloride, so bildet sich in wäßriger Lösung Aktivchlor.

Als organische Aktivchlorverbindungen kommen insbesondere die N-Chlorverbindungen in Frage, bei denen ein oder zwei Chloratome an ein Stickstoffatom gebunden sind, wobei vorzugsweise die dritte Valenz der Stickstoffatome an eine negative Gruppe führt, insbesondere an ein CO-oder $SO_2$-Gruppe. Zu diesen Verbindungen gehören Dichlor- und Trichlorcyanursäure bzw. deren Salze, chlorierte Alkylguanide oder Alkylbiguanide, chlorierte Hydantoine und chlorierte Melamine.

Als zusätzliche Hilfsstoffe werden beispielsweise aufgeführt: Als Schaumregulatoren eignen sich, vor allem bei der Verwendung von Tensiden vom Sulfonat- oder Sulfattyp, kapillaraktiver Carboxy- oder Sulfobetaine sowie die oben erwähnten Nonionics vom Alkylolamidtyp. Für diesen Zweck sind auch Fettalkohole oder höhere endständige Diole geeignet.

Ein verringertes Schäumvermögen, das vor allem beim maschinellen Waschen erwünscht ist, erreicht man vielfach durch Kombination verschiedener Tensidtypen, z.B. von Sulfaten und/oder Sulfonaten mit Nonionics und/oder mit Seifen. Bei Seifen steigt die Schaumdämpfung mit dem Sättigungsgrad und der C-Zahl des Fettsäureesters an ; Seifen der gesättigten $C_{20}$-$C_{24}$-Fettsäuren eignen sich deshalb besonders als Schaumdämpfer.

Zu den nichttensidartigen Schauminhibitoren gehören gegebenenfalls Chlor enthaltende N-alkylierte Aminotriazine, die man durch Umsetzen von 1 Mol Cyanurchlorid mit 2 bis 3 Mol eines Mono- und/oder Dialkylamins mit 6 bis 20, vorzugsweise 8 bis 18 C-Atomen im Alkylrest erhält. Ähnlich wirken propoxylierte und/oder butoxylierte Aminotriazine, z.B. Produkte, die man durch Anlagern von 5 bis 10 Mol Propylenoxid an 1 Mol Melamin und weiteres Anlagern von 10 bis 50 Mol Butylenoxid an dieses Propylenoxidderivat erhält.

Ebenfalls geeignet als nichttensidartige Schauminhibitoren sind wasserunlösliche organische Verbindungen, wie Paraffine oder Halogenparaffine mit Schmelzpunkten unterhalb von 100°C, aliphatische $C_{18}$-bis $C_{40}$-Ketone sowie aliphatische Carbonsäureester, die im Säure- oder im Alkoholrest, gegebenenfalls auch in jedem dieser beiden Reste, wenigstens 18 C-Atome enthalten (z.B. Triglyceride oder Fettsäurefettalkoholester) ; sie lassen sich vor allem bei Kombinationen von Tensiden des Sulfat- und/oder Sulfonattyps mit Seifen zum Dämpfen des Schaumes verwenden.

Die Waschmittel können optische Aufheller für Baumwolle, für Polyamid-, Polyacrylnitril- oder Polyestergewebe enthalten. Als optische Aufheller sind beispielsweise geeignet Derivate der Diaminostilbendisulfonsäure für Baumwolle, Derivate von 1,3-Diarylpyrazolinen für Polyamid, quartäre Salze von 7-Methoxy-2-benzimidazolyl-(2')-benzofuran oder von Derivaten aus der Verbindungsklasse der 7-[1',2',5'-Triazolyl-(1')]-3-[1",2",4"-triazoyl-(1")]-cumarine für Polyacrylnitril. Für Polyester geeignete Aufheller sind beispielsweise Produkte aus der Verbindungsklasse der substituierten Styrile, Ethylene, Thiophene, Naphthalindicarbonsäuren oder Derivate davon, Stilbene, Cumarine und Naphthalimide.

Als weitere Hilfsstoffe oder Formulierhilfsmittel können die dem Fachmann an sich bekannten Stoffe verwendet werden, z.B. Lösungsvermittler, wie Xylol- oder Cumolsulfonate, Stellmittel, wie Natriumsulfat, Enzyme oder Parfümöle.

Die erfindungsgemäßen Wasch- und Reinigungsmittel können z.B. pulverförmig oder flüssig sein.

Beispiel 1

Man legt 66,5 g (0,5 mol) Iminodiessigsäure in 100 g Wasser vor und stellt mit 105 g (1,05 mol) 40% iger Natronlauge einen pH-Wert von 9 ein. Anschließend tropft man innerhalb von 2 Stunden eine Lösung von 43,5

g (0,5 mol) Glycidsäureamid in 50 g Wasser bei 60°C zu.

Die vollständige Umsetzung wird nach 5-stündigem Weiterrühren bei 60°C mit Hilfe der Flüssigkeitschromatographie festgestellt. Das durch Gefriertrocknung isolierte Zwischenprodukt Isoserin-N,N-diessigsäure-monoamiddinatriumsalz hat einen Schmp. von über 300°C.

Nach Zugabe von 45 g (0,45 mol) 40% iger Natronlauge wird 4 Stunden bei 100°C gerührt, bis keine Ammoniakentwicklung mehr festgestellt wird und damit vollständige Hydrolyse der Säureamidgruppe eingetreten ist.

Es verbleiben 341 g einer ca. 40% igen gelben Lösung von 2-Hydroxy-3-aminopropionsäure-N,N-diessigsäure-trinatriumsalzt. Der Schmelzpunkt des isolierten Salzes liegt über 300°C.

Die Lösung wird mit konzentrierter Salzsäure auf einen pH-Wert von 2 eingestellt und unter Eiskühlung zum 3-fachen Volumen Methanol getropft. Nach 30-minütigem Weiterrühren bei 5°C wird der Niederschlag abfiltriert und mit 70% igem wässrigen Methanol gewaschen.

Nach dem Trocknen im Vakuum verbleiben 95 g (≙ 86% d.Th.) 2-Hydroxy-3-amino-propionsäure-N,N-diessigsäure vom Schmp. 181-184°C.

$C_7H_{11}NO_7$ (221,2)

| | | |
|---|---|---|
| ber. C 38,01% | H 5,01% | N 6,33% |
| gef. C 37,92% | H 5,28% | N 6,15% |

Beispiel 2

Man legt 66,5 g (0,5 mol) Iminodiessigsäure in 100 g Wasser vor und stellt mit 100 g (1 mol) 40% iger Natronlauge einen pH-Wert von 8,5 ein. Bei 40 bis 45°C wird anschließend eine Lösung von 59,5 g (0,5 mol) Natriumglycidat-hemihydrat in 100 ml Wasser innerhalb von 1 Stunde zugetropft. Nach 3-stündigem Weiterrühren bei 45°C wird chromatographisch das Ende der Reaktion festgestellt.

Es liegt eine wässrige Lösung von 2-Hydroxy-3-amino-propionsäure-N,N-diessigsäure-trinatriumsalz vor. Die Überführung in die freie Säure erfolgte analog zu Beispiel 1. Ausbeute : 75% d.Th..

Beispiel 3

A : Herstellung von 2-Hydroxy-3-amino-propionsäure :

Zu einer Lösung von 119 g (1 mol) Natriumglycidat-hemihydrat in 150 g Wasser werden bei 50°C in 1 Stunde 85 g (1,25 mol) konzentrierter, 25 Gew.% iger wäßriger Ammoniak zugetropft. Anschließend wird 5 Stunden bei 50°C nachgerührt.

Man entfernt nicht umgesetzten Ammoniak und einen Teil des Lösemittels unter vermindertem Druck (Wasserstrahlpumpe) und stellt mit Eisessig einen pH-Wert von 5 ein. Der ausgefallene Niederschlag wird abfiltriert und mit Methanol gewaschen.

B : Umsetzung mit Formaldehyd und Blausäure

Die nach dem Trocknen erhaltenen 85 g 2-Hydroxy-3-amino-propionsäure (≙ 81% d. Th.) (0,81 mol) werden in 200 g Wasser aufgenommen und mit 40% iger Natronlauge wird ein pH-Wert von 8,5 eingestellt. Bei 15 bis 20°C werden innerhalb von 2 Stunden gleichzeitig 43,7 g (1,62 mol) flüssige Blausäure und 165 g (1,65 mol) 30% iger Formaldehyd zugetropft.

Nach 2,5-stündigem Weiterrühren bei 25°C resultiert eine wässrige Lösung von 2-Hydroxy-3-amino-propionsäure-N,N-diacetonitril.

Diese wird mit 1,65 mol 40% iger Natronlauge bei 100°C verseift. Nach 3,5 Stunden war aller Ammoniak ausgetrieben.

Aus der resultierenden orangenen Lösung von 2-Hydroxy-3-amino-propionsäure-N,N-diessigsäure-trinatriumsalz wird wie in Beispiel 1 die freie Säure in einer Ausbeute von 89% isoliert.

Beispiel 4

Die gemäß Beispiel 3A erhaltene Lösung wird ohne Isolierung der 2-Hydroxy-3-amino-propionsäure und ohne Ansäuern mit Eisessig nach Entfernung des überschüssigen Ammoniaks gemäß Beispiel 3B mit Formaldehyd und Blausäure umgesetzt.

Nach der alkalischen Verseifung resultiert eine orangene Lösung von 2-Hydroxy-3-amino-propionsäure-N,N-diessigsäure-trinatriumsalz, aus welcher die freie Säure analog zu Beispiel 1 in einer 74% igen Gesamtausbeute isoliert wird.

Beispiel 5

Beispiel 3 wird analog mit 1 Mol Glycidylamid anstelle von Natriumglycidat-hemihydrat wiederholt.

Die 2-Hydroxy-3-amino-propionsäure als Zwischenprodukt wurde hier in 76% iger Ausbeute isoliert und gemäß 3B zur 2-Hydroxy-3-aminopropionsäure-N,N-diessigsäure weiter umgesetzt.

Beispiel 6

Zu einer Lösung von 87 g (1,0 mol) Glycidsäureamid in 260 g Wasser tropft man innerhalb von 1,5 Stunden 590 g einer 30% igen wäßrigen Lösung von Iminodiessigsäure-di-natrium (1,0 mol) bei 55°C unter kräftigem Rühren zu.

Nach 4-stündigem Weiterrühren bei 70°C werden 103 g (1,03 mol) 40% ige Natronlauge zugesetzt und man erhitzt 3,5 Stunden auf 100 bis 105°C, bis keine Ammoniakentwicklung mehr festgestellt wird. Es liegt anschließend eine wäßrige Lösung von 2-Hydroxy-3-amino-propionsäure-N,N-diessigsäure-trinatriumsalz vor, aus der die freie Tricarbonsäure analog Beispiel 1 in 87% iger Ausbeute isoliert wird.

Beispiel 7

0,2 mol in 50 ml Wasser gelöste und nach Beispiel 3a) hergestellte 2-Hydroxy-3-aminopropionsäure werden mit 10 gew.% iger Natronlauge auf einen pH-Wert von 11,5 eingestellt und 0,4 mol in Wasser gelöste und mit 10 gew.% iger Natronlauge auf pH 10 neutralisierte Chloressigsäure so zugetropft, daß der pH-Wert mit 40 gew.% iger Natronlauge zwischen pH 9 und 10 gehalten werden kann.

Es wird 12 Stunden bei 100°C nachgerührt und die Lösung nach dem Abkühlen mit konzentrierter Salzsäure auf einen pH-Wert von 2 gebracht.

Unter Eiskühlung tropft man das 3-fache Volumen Methanol zu, rührt ca. 30 Minuten nach, filtriert den entstandenen Niederschlag ab und wäscht mit 70 gew.% igem Methanol nach. Nach dem Trocknen verbleiben 40,2g ($\hat{=}$ 91% d.Th.) 2-Hydroxy-3-amino-propionsäure-N,N-diessigsäure.

Die aus der freien 2-Hydroxy-3-amino-propionsäure-N,N-diessigsäure erhaltenen Trikalium- und Triammoniumsalze wiesen Schmelzpunkte von jeweils über 300°C auf.

Anwendungstechnische Eigenschaften

I. Bestimmung der Eisenkomplexierkapazität

Die inhibierende Wirkung von Komplexbildnern auf die Ausfällung von Eisen(III)-hydroxid wird durch Trübungstitration bestimmt. Es wird die zu untersuchende Wirksubstanz (W.S.) vorgelegt und in alkalischer Lösung mit Eisen(III)-chlorid-Lösung bis zur Trübung titriert.

Die Titration erfolgt automatisch mit Hilfe eines Titroprozessors ; es wird dabei die Lichtdurchlässigkeit der Lösung mit einem Lichtleiterphotometer verfolgt. Der Endpunkt der Titration wird durch das Auftreten einer Trübung angezeigt. Er gibt die Menge an gebundenem Eisen an und ist ein Maß für die Stärke des gebildeten Komplexes im Vergleich zu Eisenhydroxid.

Bei Verbindungen mit einer dispergierenden Wirkung gegenüber Eisenhydroxid tritt meist vor dem Erreichen des Endpunktes eine Verfärbung ein.

Das Ausmaß der Verfärbung (hervorgerufen durch kolloidal dispergiertes Eisenhydroxid) gibt einen Hinweis auf die Diisozationsneigung des gebildeten Komplexes. Ein grobes Maß dafür ist die Steigung der Titrationskurve vor Erreichen des Äquivalenzpunktes. Sie wird gemessen in % Transmission/ml $FeCl_3$-Lösung. Die reziproken Werte geben somit die Stärke der Komplexe an.

Ausführungsvorschrift :

1 mmol der zu untersuchenden Substanz (W.S.) wird in 100 ml dest. $H_2O$ gelöst. Der pH-Wert wird mit 1n NaOH auf 10 eingestellt und während der Titration konstant gehalten. Man titriert bei Raumtemperatur mit einer Geschwindigkeit von 0,4 ml/min. mit 0,05 m $FeCl_3$-Lösung.

Die Komplexierkapazität wird ausgedrückt als :

$$\text{mol Fe/mol W.S.} = \frac{\text{ml verbrauchter } FeCl_3\text{-Lsg.}}{20}$$

oder

$$\text{mg Fe/g W.S.} = \frac{\text{ml verbrauchter } FeCl_3\text{-Lsg}}{MG_{W.S.}} \times 2790$$

II. Prüfung der Natriumperboratstabilisierung in Waschflotten

Prinzip

Das für die Bleichwirkung in natriumperborathaltigen Waschmittelformulierungen verantwortliche Wasserstoffperoxid wird durch Schwermetallionen (Fe, Cu, Mn) katalytisch zersetzt. Durch Komplexierung der Schwermetallionen läßt sich dies verhindern. Die peroxidstabilisierende Wirkung der Komplexbildner wird über den Restperoxidgehalt nach Warmlagerung einer schwermetallhaltigen Waschflotte geprüft.

Der Gehalt an Wasserstoffperoxid wird vor und nach der Lagerung durch eine Titration mit Kaliumpermanganat in saurer Lösung bestimmt.

Zur Prüfung auf Perboratstabilisierung werden zwei Waschmittelformulierungen benutzt, wobei die Zersetzung bei der Warmlagerung durch Zugabe von Schwermetallkatalysatoren (2,5 ppm Mischung aus 2 ppm $Fe^{3+}$, 0,25 ppm $Cu^{2+}$, 0,25 ppm $Mn^{2+}$) erfolgt.

1. Phosphathaltige Formulierung Zusammensetzung (in Gew.%) :

19,3% Natrium-$C_{12}$-Alkylbenzolsulfonat (50 gew.% ige wäßrige Lösung)
15,4% Natriumperborat · 4 $H_2O$
30,8% Natriumtriphosphat
2,6% Copolymer aus Maleinsäure und Acrylsäure (50 : 50, mittleres MG 50000)
31,0% Natriumsulfat, wasserfrei
0,9% erfindungsgemäßer Komplexbildner oder Vergleichsverbindung

Die Waschmittelkonzentration beträgt 6,5 g/l unter Verwendung von Wasser mit 25° dH. Die Lagerung erfolgt bei 80°C 2 Stunden lang.

2. Phosphatreduzierte Formulierung Zusammensetzung (in Gew.%) :

15% Natrium-$C_{12}$-Alkylbenzolsulfonat (50 gew.% ige wäßrige Lösung)
5% Anlagerungsprodukt von 11 Mol Ethylenoxid an 1 Mol Talgfettalkohol
20% Natriumperborat · 4 $H_2O$
6% Natrium-metasilikat · 5 $H_2O$
1,25% Magnesiumsilikat
20% Natriumtriphosphat
31,75% Natriumsulfat, waserfrei
1% erfindungsgemäßer Komplexbildner oder Vergleichsverbindung

Die Waschmittelkonzentration beträgt 8 g/l unter Verwendung von Wasser mit 25° dH. Die Lagerung erfolgt bei 60°C 1 Stunde lang.

III Bestimmung des Calcium-Bindevermögens :

Meßprinzip

Die inhibierende Wirkung von Komplexbildnern oder Dispergiermitteln auf die Ausfällung von Calciumcarbonat wird durch Trübungstitration bestimmt. Es wird die zu untersuchende Substanz vorgelegt und in Gegenwart von Natriumcarbonat mit Calciumacetatlösung titriert. Der Endpunkt wird durch Bildung des Calciumcarbonat-Niederschlages angezeigt. Durch Verwendung einer ausreichenden Menge an Natriumcarbonat wird sichergestellt, daß die Messung auch dann ein korrektes Ergebnis liefert, wenn die Wirkung nicht nur auf einer Komplexierung der Calciumionen beruht, sondern auf der Dispergierung von Calciumcarbonat. Werden nämlich zu kleine Natriumcarbonatmengen eingesetzt, besteht die Gefahr, daß das Dispergiervermögen des Produktes nicht ausgeschöpft wird ; in diesem Fall wird der Titrationsendpunkt durch die Fällung des Calcium-Salzes der untersuchten Verbindung bestimmt.

Während der Titration wird die Änderung der Lichtdurchlässigkeit mit Hilfe eines Lichtleiterphotometers verfolgt. Bei letzterem wird ein über Glasfaser in die Lösung geleiteter Lichtstrahl an einem Spiegel reflektiert und die Intensität des reflektierten Lichts gemessen.

Reagenzien :

0,25 m $Ca(OAc)_2$-Lösung
10% ige $Na_2CO_3$-Lösung
1 n NaOH-Lösung
1% ige Salzsäure

Durchführung :

1 g W.S. in Form des Trinatriumsalzes wird in 100 ml dest. $H_2O$ gelöst. Anschließend werden 10 ml 10% ige $Na_2CO_3$-Lösung zugegeben. Bei Raumtemperatur (RT) und einem während der Titration konstant gehalten pH-Wert von 11 und bei 80°C mit einem pH-Wert von 10 wird mit 0,25 m $Ca(OAc)_2$-Lösung kontinuierlich mit 0,2 ml/min automatisch titriert.

Berechnung :

Menge mg $CaCO_3$/gW.S. = Verbrauch an $Ca(OAc)_2$-Lösung in ml × 25. Bei der automatischer Titration ist der 1. Knickpunkt der Titrationskurve der Endpunkt.

Die erhaltenen Ergebnisse sind in Tabelle 1 zusammengestellt :

Tabelle 1

| | Calcium-Bindevermögen mg $CaCO_3$/g W.S. | | Eisen-Bindevermögen | | | Perborat-stabilisierung in [%] Waschmittel-formulierung | |
|---|---|---|---|---|---|---|---|
| | RT/ pH 11 | 80°C/ pH 10 | mol $Fe^{3+}$ / mol W.S. | mg$Fe^{3+}$ / g W.S. | %Transmission (am Knickpunkt) / ml/$FeCl_3$ | 1 | 2 |
| 2-Hydroxy-3-aminopropionsäure-N,N-diessigsäure-$Na_3$ | 275 | 200 | 0,6 | 113 | 39 | 43,3 | 82,0 |
| Na-triphosphat | 215 | 150 | | | | | |
| $NTA-Na_3$ | 350 | 250 | 0,25 | 54 | 11 | 24,5 | 32,5 |
| $EDTA-Na_4$ | 275 | 240 | 0,30 | 44 | 1,2 | 20 | 34,0 |

Aus den Ergebnissen folgt, daß das Calcium-Bindevermögen, insbesondere bei Raumtemperatur, vergleichbar ist mit dem Natriumsalz von EDTA und wesentlich besser als das des Natriumtriphosphats ist. Bei dem hohen Wert für das Natriumsalz des NTA sollte das kleinere Molekulargewicht mit berücksichtigt werden. Das Bindevermögen für Eisen ist gegenüber NTA und EDTA praktisch doppelt so hoch. Die Stärke des gebildeten Komplexes, ausgedrückt in % Transmission/ml $FeCl_3$-Lösung, ist um ein Vielfaches größer als beim Komplex der Ethylendiamintetraessigsäure und der Nitrilotriessigsäure.

Der besonders überraschende Effekt liegt in der hervorragenden Perboratstabilisierung für die erfindungsgemäße N-haltige und relative niedermolekulare Verbindung.

Bei einer Verwendung als Gerüstsubstanz werden mit Standardwaschmittelformulierungen gute Waschergebnisse, insbesondere im Hinblick auf die Inkrustationisinhibierung, gemessen am Aschegehalt, erhalten.

## Ansprüche

1. 2-Hydroxy-3-aminopropionsäure-N,N-diessigsäure und ihre Derivative der Formel I

$$(Y-CH_2)_2NCH_2\underset{\underset{OH}{|}}{C}H-(COX) \qquad (I)$$

in der Y für den Rest –COOH, der gegebenenfalls in Form eines Alkalimetall-, Ammonium- oder substituierten Ammoniumsalzes vorliegt, für einen Rest –COOR$^1$, in dem R$^1$ einen Alkyrest mit 1 bis 4 C-Atomen bedeutet, oder für den Rest -CN steht und
X eine Hydroxylgruppe, wobei die dadurch vorliegende Carbonsäuregruppe gegebenenfalls in Form eines Alkalimetall-, Ammonium- oder substituierten Ammoniumsalzes vorliegt, einen Rest –OR$^2$, in dem R$^2$ für einen Alkylrest mit 1 bis 4 C-Atomen steht, oder einen Rest –NR$^3$R$^4$, in dem R$^3$ und R$^4$ gleich oder verschieden sind und für ein Wasserstoffatom oder einen Alkylrest mit 1 bis 4 C-Atomen stehen,
bedeutet.

2. Komplexbildner für Schwermetall- und Erdalkaliionen der Formel I nach Anspruch 1, in der Y den Rest –COOH und X –OH bedeuten, wobei die vorliegenden Carbonsäuregruppen gegebenenfalls in Form der Alkalimetall-, Ammonium- oder substituierten Ammoniumsalze vorliegen.

3. Verfahren zur Herstellung von Verbindungen der Formel I nach Anspruch 1 oder 2, dadurch gekennzeichnet, daß man eine Verbindung der Formel II

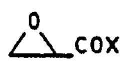

(II)

in der X die für Formel I angegebenen Bedeutungen aufweist, mit einer Verbindung der Formel III

$$HN(CH_2-Y)_2 \quad (III)$$

in der Y die für Formel I angegebenen Bedeutungen aufweist, in Wasser, einem organischen Lösungsmittel oder ihren Mischungen bei Temperaturen von 10 bis 80°C umsetzt und gegebenenfalls die vorliegenden Amid-, Ester- und Nitrilgruppen in Gegenwart von einer Säure oder Base hydrolysiert und gegebenenfalls die freie Säure oder ein Salz gemäß Formel I isoliert.

4. Verfahren zur Herstellung von Verbindungen der Formel I nach Anspruch 1 oder 2, dadurch gekennzeichnet, daß man 1 Mol 2-Hydroxy-3-aminopropionsäure gegebenenfalls in Form eines Alkalisalzes, eines Alkylesters mit 1 bis 4 C-Atomen im Alkyl oder des Säureamids, gegebenenfalls am Amidstickstoffatom durch ein oder zwei Alkylreste mit 1 bis 4 C-Atomen substituiert, in Wasser, einem organischen Lösungsmittel oder ihren Mischungen bei Temperaturen von 0 bis 100°C mit 2,0 bis 2,6 Mol einer Monohalogenessigsäure, einem Monohalogenessigsäurealkylester mit 1 bis 4 C-Atomen im Alkyl oder Monohalogenacetonitril in alkalischem Medium oder in Gegenwart eines Säurefängers umsetzt und gegebenenfalls die vorliegenden Amid-, Ester- und Nitrilgruppen in Gegenwart von einer Säure oder Base hydrolysiert und gegebenenfalls die freie Säure oder ein Salz gemäß Formel I isoliert.

5. Verfahren zur Herstellung von Verbindungen der Formel I nach Anspruch 1 oder 2, in der Y die für Formel I angegebenen Bedeutungen außer dem Rest –COOR$^1$ und X die für Formel I angegebenen Bedeutungen aufweist, dadurch gekennzeichnet, daß man 1 Mol 2-Hydroxy-3-aminopropionsäure gegebenenfalls in Form eines Alkalisalzes, eines Alkylesters mit 1 bis 4 C-Atomen im Alkyl oder des Säureamids, gegebenenfalls am Amidstickstoffatom durch ein oder zwei Alkylreste mit 1 bis 4 C-Atomen substituiert, in Wasser, einem organischen Lösungsmittel oder ihren Mischungen mit 2,0 bis 2,6 Mol Formaldehyd und 2,0 bis 2,3 Mol flüssiger Blausäure bei Temperaturen von 0 bis 45°C oder 2 bis 2,3 Mol Alkalicyanid bei Temperaturen von 40 bis 100°C umsetzt und gegebenenfalls die vorliegenden Amid-, Ester- und Nitrilgruppen in Gegenwart von einer Säure oder Base hydrolysiert und gegebenenfalls die freie Säure oder ein Salz gemäß Formel I isoliert.

6. Verwendung von Verbindungen der Formel (I) nach Anspruch 2 als Komplexbildner für Schwermetall- und/oder Erdalkaliionen.

7. Verwendung von Verbindungen der Formel (I) nach Anspruch 2 als Gerüststoff in Wasch- und Reinigungsmitteln.

8. Verwendung von Verbindungen der Formel (I) nach Anspruch 2 in Wasch- und Reinigungsmitteln als Bleichmittelstabilisatoren.

9. Wasch- und Reinigungsmittel, enthaltend eine Verbindung der Formel (I) nach Anspruch 2 in einer Menge von 0,01 bis 20 Gew.%, bezogen auf das Gesamtgewicht.

10. Verwendung von Verbindungen der Formel I, in denen Y einen Rest $-COOR^1$, in dem $R^1$ einen Alkylrest mit 1 bis 4 C-Atomen oder den Rest $-CN$ und X einen Rest $-OR^2$, in dem $R^2$ für einen Alkylrest mit 1 bis 4 C-Atomen oder einen Rest $-NR^3R^4$, in dem $R^3$ und $R^4$ gleich oder verschieden sind und für ein Wasserstoffatom oder einen Alkylrest mit 1 bis 4 C-Atomen stehen, bedeuten, als Zwischen produkte zur Herstellung von Verbindungen der Formel I nach Anspruch 2.

11. Verbindungen der Formel I, in denen Y einen Rest $-COOR^1$, in dem $R^1$ einen Alkylrest mit 1 bis 4 C-Atomen oder den Rest $-CN$ und X einen Rest $-OR^2$, in dem $R^2$ für einen Alkylrest mit 1 bis 4 C-Atomen odereinen Rest $-NR^3R^4$, in dem $R^3$ und $R^4$ gleich oder verschieden sind und für ein Wasserstoffatom oder einen Alkylrest mit 1 bis 4 C-Atomen stehen, bedeuten, als Zwischenprodukte zur Herstellung von Verbindungen der Formel I nach Anspruch 2.

## Claims

1. 2-hydroxy-3-aminopropionic-N,N-diacetic acid, or a derivative thereof, of the formula I

$$(Y{-}CH_2)_2NCH_2\underset{\underset{OH}{|}}{C}H{-}(COX) \qquad (I)$$

where Y is a $-COOH$ radical, which may be present in the form of an alkali metal, ammonium or substituted ammonium salt, a $-COOR^1$ radical where $R^1$ is alkyl of 1 to 4 carbon atoms, or a $-CN$ radical, and X is hydroxyl, in which case the then resulting carboxyl may be present in the form of an alkali metal, ammonium or substituted ammonium salt, an $-OR^2$ radical where $R^2$ is alkyl of 1 to 4 carbon atoms, or an $-NR^3R^4$ radical where $R^3$ and $R^4$ are identical or different and each is hydrogen or alkyl of 1 to 4 carbon atoms.

2. A heavy metal and alkaline earth metal ion complexing agent of the formula I as claimed in claim 1 where Y is $-COOH$ and X is $-OH$, and the carboxyl groups present may be in the form of an alkali metal, ammonium or substituted ammonium salt.

3. A process for preparing a compound of the formula I as claimed in claim 1 or 2, which comprises reacting a compound of the formula II

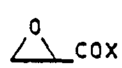

(II)

where X has the meanings indicated for the formula I, with a compound of the formula III

$$HN(CH_2-Y)_2 \quad (III)$$

where Y has the meanings indicated for the formula I, in water, in an organic solvent or in a mixture thereof at from 10 to 80°C and hydrolyzing any amide, ester and nitrile group present in the presence of an acid or base and as desired isolating the free acid or a salt conforming to the formula I.

4. A process for preparing a compound of the formula I as claimed in claim 1 or 2, which comprises reacting 1 mole of 2-hydroxy-3-aminopropionic acid, if desired in the form of an alkali metal salt, of an alkyl ester of 1 to 4 carbon atoms in the alkyl or of the amide, unsubstituted or mono- or disubstituted on the amide nitrogen by alkyl of 1 to 4 carbon atoms, in water, in an organic solvent or in a mixture thereof at from 0 to 100°C with from 2.0 to 2.6 moles of a monohaloacetic acid, an alkyl monohaloacetate of 1 to 4 carbon atoms in the alkyl or monohaloacetonitrile in an alkaline medium or in the presence of an acid acceptor and hydrolyzing any amide, ester and nitrile groups present in the presence of an acid or base and as desired isolating the free acid or a salt conforming to the formula I.

5. A process for preparing a compound of the formula I as claimed in claim 1 or 2, where Y has the meanings indicated for the formula I other than $-COOR^1$ and X has the meanings indicated for the formula I, which comprises reacting 1 mole of 2-hydroxy-3-aminopropionic acid, if desired in the form of an alkali metal salt, of an alkyl ester of 1 to 4 carbon atoms in the alkyl or of the amide, unsubstituted or mono- or disubstituted on the amide nitrogen by alkyl of 1 to 4 carbon atoms, in water, in an organic solvent or in a mixture thereof with from 2.0 to 2.6 moles of formaldehyde and from 2.0 to 2.3 moles of liquid hydrocyanic acid at from 0 to 45°C or with from 2 to 2.3 moles of an alkali metal cyanide at from 40 to 100°C and hydrolyzing any amide, ester and nitrile groups present in the presence of an acid or base and as desired isolating the free acid or a salt conforming to the formula I.

6. Use of compounds of the formula (I) as claimed in claim 2 as complexing agents for heavy metal and/or alkaline earth metal ions.

7. Use of compounds of the formula (I) as claimed in claim 2 as builders in detergents.

8. Use of compounds of the formula (I) as claimed in claim 2 in detergents as bleaching agent stabilizers.

9. A detergent containing a compound of the formula (I) as claimed in claim 2 in an amount from 0.01 to 20% by weight, based on the total weight.

10. Use of compounds of the formula I where Y is $-COOR^1$, where $R^1$ is alkyl of 1 to 4 carbon atoms, or $-CN$, and X is $-OR^2$, where $R^2$ is alkyl of 1 to 4 carbon atoms, or $-NR^3R^4$, where $R^3$ and $R^4$ are identical or different and each is hydrogen or alkyl of 1 to 4 carbon atoms, as an intermediate for preparing a compound of the formula I as claimed in claim 2.

11. A compound of the formula I where Y is $-COOR^1$, where $R^1$ is alkyl of 1 to 4 carbon atoms, or $-CN$, and X is $-OR^2$, where $R^2$ is alkyl of 1 to 4 carbon atoms, or $-NR^3R^4$, where $R^3$ and $R^4$ are identical or different and each is hydrogen or alkyl of 1 to 4 carbon atoms, as an intermediate for preparing a compound of the formula I as claimed in claim 2.

**Revendications**

1. Acide 2-hydroxy-3-aminopropionique-N,N-diacétique et leurs dérivés de la formule

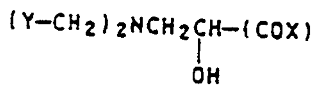

(I)

dans laquelle Y représente le radical $-COOH$, qui se présente éventuellement sous la forme d'un sel de métal alcalin, d'ammonium ou d'ammonium substitué, un radical $-COOR^1$ où $R^1$ représente un groupe alkyle qui comporte de 1 à 4 atomes de carbone, ou le radical $-CN$ et
X représente un radical hydroxyle, auquel cas le radical acide carboxylique qui existe dans ce cas se présente éventuellement sous la forme d'un sel de métal alcalin, d'ammonium ou d'ammonium substitué, un radical $-OR^2$ où $R^2$ représente un radical alkyle qui comporte de 1 à 4 atomes de carbone, ou un radical $-NR^3R^4$ où $R^3$ et $R^4$ sont identiques ou différents et représentent chacun un atome d'hydrogène ou un radical alkyle qui comporte de 1 à 4 atomes de carbone.

2. Agents formateurs de complexes pour des ions de métaux lourds et de métaux alcalino-terreux, de la formule I selon la revendication 1, dans laquelle Y représente le radical $-COOH$ et X représente le radical $-OH$, où les radicaux carboxyle qui existent se présentent éventuellement sous la forme des sels de métaux alcalins, d'ammonium ou d'ammonium substitué.

3. Procédé de préparation de composés de la formule I selon la revendication 1 ou 2, caractérisé en ce que l'on fait réagir un composé de la formule II

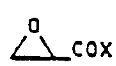

(II)

dans laquelle X possède les significations qui lui ont été attribuées à propos de la définition de la formule I, avec un composé de la formule III

$$HN(CH_2-Y)_2 \quad (III)$$

dans laquelle Y possède les significations qui lui ont été attribuées à propos de la définition de la formule I,

dans de l'eau, un solvant organique, ou leurs mélanges, à des températures de 10 à 80°C et on hydrolyse éventuellement les radicaux amide, ester et nitrile présents en présence d'un acide ou d'une base et on isole éventuellement l'acide libre ou un sel selon la figure 1.

4. Procédé de préparation de composés de la formule I selon la revendication 1 ou 2, caractérisé en ce que l'on fait réagir une mole d'acide 2-hydroxy-3-aminopropionique, éventuellement sous forme d'un sel de métal alcalin, d'un ester alkylique dont le radical alkyle comporte de 1 à 4 atomes de carbone ou de l'amide d'acide, éventuellement substitué sur l'atome d'azote amidique par un ou deux radicaux alkyle qui comportent de 1 à 4 atomes de carbone, dans l'eau, un solvant organique ou leurs mélanges, à des températures de 0 à 100°C, avec 2,0 à 2,6 moles d'un acide monohalogénoacétique, d'un monohalogénoacétate d'alkyle dont le radical alkyle comporte de 1 à 4 atomes de carbone, ou d'un monohalogénoacétnotrile, en milieu alcalin, ou en présence d'un agent fixateur d'acides et on hydrolyse éventuellement les radicaux amide, ester et nitrile présents en présence d'un acide ou d'une base et on isole éventuellement l'acide libre ou un sel selon la formule I.

5. Procédé de préparation de composés de la formule I selon la revendication 1 ou 2, dans laquelle Y possède les significations qui lui ont été attribuées à propos de la définition de la formule I à l'exception du radical $-COOR^1$ et X possède les significations qui lui ont été attribuées à propos de la définition de la formule I, caractérisé en ce que l'on fait réagir une mole d'acide 2-hydroxy-3-aminopropionique, éventuellement sous la forme d'un sel de métal alcalin, d'un ester alkylique dont le radical alkyle comporte de 1 à 4 atomes de carbone, ou de l'amide d'acide, éventuellement substitué sur l'atome d'azote amidique par un ou deux radicaux alkyle qui comportent de 1 à 4 atomes de carbone, dans l'eau, un solvant organique, ou leurs mélanges, avec 2,0 à 2,6 moles de formaldéhyde et 2,0 à 2,3 moles d'acide cyanhydrique liquide, à des températures de 0 à 45°C, ou 2 à 2,3 moles d'un cyanure de métal alcalin, à des températures de 40 à 100°C et on hydrolyse éventuellement les radicaux amide, ester et nitrile présents, en présence d'un acide ou d'une base et on isole éventuellement l'acide libre ou un sel selon la formule I.

6. Utilisation des composés de la formule (I) selon la revendication 2, à titre d'agents formateurs de complexes pour les ions de métaux lourds et/ou de métaux alcalino-terreux.

7. Utilisation des composés de la formule (I) selon la revendication 2, à titre d'adjuvants actifs dans des détergents et agents de nettoyage ou lavage ou lessivage.

8. Utilisation des composés de la formule (I) selon la revendication 2 dans des détergents et des agents de nettoyage, lavage ou lessivage, à titre de stabilisateurs d'agents de blanchiment.

9. Détergents et agents de lavage, nettoyage ou lessivage, qui contiennent un composé de la formule (I) selon la revendication 2 en une proportion de 0,01 à 20% en poids, par rapport au poids total.

10. Utilisation de composés de la formule I dans laquelle Y représente un radical $-COOR^1$, où $R^1$ représente un radical alkyle qui comporte de 1 à 4 atomes de carbone, ou le radical $-CN$ et X représente un radical $-OR^2$, où $R^2$ représente un radical alkyle qui comporte de 1 à 4 atomes de carbone, ou un radical $-NR^3R^4$, où $R^3$ et $R^4$ sont identiques ou différents et représentent chacun un atome d'hydrogène ou un radical alkyle qui comporte de 1 à 4 atomes de carbone, à titre de produits intermédiaires pour la préparation de composés de la formule I selon la revendication 2.

11. Composés de la formule I dans laquelle Y représente un radical $-COOR^1$, où $R^1$ représente un radical alkyle qui comporte de 1 à 4 atomes de carbone, ou le radical $-CN$ et X représente un radical $OR^2$, où $R^2$ représente un radical alkyle qui comporte de 1 à 4 atomes de carbone, ou un radical $-NR^3R^4$, où $R^3$ et $R^4$ sont identiques ou différents et représentent chacun un atome d'hydrogène ou un radical alkyle qui comporte de 1 à 4 atomes de carbone, à titre de produits intermédiaires pour la préparation de composés de la formule I selon la revendication 2.